# EUROPÄISCHE PATENTANMELDUNG

(11) **EP 0 880 952 A1**
(43) Veröffentlichungstag der Anmeldung: **02.12.1998**
(21) Anmeldenummer: 98104194.0
(22) Anmeldetag: 09.03.1998
(51) Int. Cl.: A61F 7/10, A63H 3/00

(54) **Spielfigur**

(30) Priorität: 28.05.1997 DE 29709397 U
(71) Anmelder: Top-Idee Geschenk-Service GmbH, 91154 Roth (DE)
(72) Erfinder: Becka, Martina, 81479 München (DE)
(74) Vertreter: Grünecker, Kinkeldey, Stockmair & Schwanhäusser Anwaltssozietät

(57) **Zusammenfassung**

Es wird eine Spielfigur beschrieben, die sich sowohl zum Spielen als auch gleichzeitig zum wirksamen Kühlen eignet. Zu diesem Zweck weist die Spielfigur, die insbesondere eine Plüschfigur ist, einen mit Polstermaterial (4) gefüllten Körper (2), ein im Körper (2) untergebrachtes Kühlelement (7) und ein im wesentlichen polstermaterialfreies Kühlfenster (8) auf, das an einer Seite des Kühlelements (7) vorgesehen ist.

## Beschreibung

Die Erfindung bezieht sich auf eine Spielfigur, insbesondere eine Plüschfigur.

Spielfiguren, insbesondere aus Plüsch, sind als Kinderspielzeug weit verbreitet. Es ist weiterhin, beispielsweise aus dem DE-GM 88 01 025 bekannt, in eine Spielfigur beispielsweise eine Wärmeeinrichtung einzusetzen, um die Spielfigur gleichzeitig als Wärmflasche verwenden zu können Aus der US-PS 4 204 110 ist eine Figur in Form eines Bären bekannt, deren Rumpf durch eine mit Wasser gefüllte Base gebildet wird, in der eine elektrische Heizung angeordnet ist. Die Base kann jedoch auch mit Eiswürfel und kaltem Wasser gefüllt und zum Kühlen verwendet werden. Die bekannte Figur eignet sich jedoch nicht zum Spielen, da einerseits die Gefahr besteht, daß das Wasser ausläuft und da sie sich andererseits wegen der Wasserfüllung schwer und wenig einladend zum Spielen anfühlt. Aus dem DE-GM 91 07 873 ist es weiterhin bekannt, eine Spielfigur, insbesondere eine Plüschfigur als dekorative Halterung für die verschiedensten Behälter auszubilden.

In allen Fällen sind jedoch die aufzunehmenden Gegenstände im Polstermaterial im wesentlichen vollkommen versenkt.

Aus der US-PS 4 714 445 ist ein Spielzeug, dargestellt ist ein Teddybär, mit Heizeinlage bekannt, wobei die Heizeinlage direkt unterhalb der äußeren Hülle angeordnet ist. Die Heizeinlage besteht aus einer mit Flüssigkeit gefüllten Base und ist auswechselbar, damit die Flüssigkeit wieder aufgewärmt werden kann. Die Blase befindet sich nicht unter allen Bereichen des Spielzeuges, wobei beim Bär die Extremitäten, die Schnauze und die Ohren nicht beheizt werden. Die Heizeinlage befindet sich somit im Rumpf und am Hinterkopf, d.h. in allen denjenigen Teilen, die das Kind ergreift, wenn es den Bären an sich drückt. Zwar wird in dieser Druckschrift erwähnt, daß die Base auch gekühlt werden kann, der Zweck eines gekühlten Bären ist jedoch nicht erwähnt. Auf jeden Fall ist nicht zu erwarten, daß ein gekühlter Bär sowohl als "Tröster" als auch zum Kühlen von Beulen und dgl. eingesetzt werden kann, da der vollständig gekühlte Bär nicht dazu einlädt, daß ihn das Kind an sich drückt.

Aus der US-PS 4 816 000 ist ein Überzug für eine der üblichen Gummiwärmflaschen bekannt, die je nach Bedarf entweder mit kaltem oder mit warmem Wasser gefüllt werden können. Der Überzug ist als Tierkörper ausgebildet, wobei die Extremitäten, der Schwanz und der Kopf nicht von der Wärmflasche ausgefüllt werden. Die üblichen Wärmflaschen sind jedoch, in gefülltem Zustand, viel zu schwer zu Spielen und weder als "Tröster" einzusetzen noch geeignet, beispielsweise auf Beulen gelegt zu werden.

Die EP-A-275 167 zeigt eine Spielfigur mit einer elektrischen Heizeinrichtung, die im Inneren von Polstermaterial untergebracht ist und die von ihr erzeugte Wärme auf eine flexible Platte an der Außenseite der Spielfigur überträgt. Die Spielfigur ist somit nur zur Wärmeübertragung und nicht zum Kühlen geeignet.

Auch die US-PS 1 558 278 beschreibt eine Spielfigur, die nur zum Heizen ausgebildet ist und bei der die Heizeinrichtung allseits mit Polstermaterial umgeben ist.

Allseits mit Polstermaterial umgeben ist auch das Heiz- oder Kühlelement der Spielfigur nach der US-PS 4 694 829.

Die US-PS 2 538 123 beschreibt einen Überzug für die üblichen Gummiwärmflaschen in Form einer Puppe.

Die US-PS 5 002 511 beschreibt eine Spielfigur mit einer unter dem Überzug angeordneten Schicht für fernes Infrarot, die bei dem die Spielfigur haltenden Kind einen Wärmeeindruck erzeugen soll, der dem Eindruck entspricht, den ein lebendes Tier hinterläßt.

Der Erfindung liegt die Aufgabe zugrunde, eine Spielfigur bereitzustellen, die sich sowohl zum Spielen als auch gleichzeitig zum wirksamen Kühlen eignet.

Die Aufgabe wird durch die im Anspruch 1 angegebenen Merkmale gelöst.

Durch die erfindungsgemäße Ausgestaltung kann die Spielfigur sowohl als Spielzeug als auch als "Trösten" der Kinder bei Fieber, kleineren Verletzungen oder Beulen eingesetzt werden, indem ein im Kühlschrank bereitgehaltenes oder schnell gekühltes Kühlelement in die Spielfigur eingesetzt wird, und zusammen mit der Spielfigur an die Verletzung und/oder die Beule gedrückt wird. Durch das im Körper der Spielfigur vorhandenen, im wesentlichen polstermaterialfreie Kühlfenster kann die Kälte des Kühlelementes unmittelbar und örtlich begrenzt nach außen treten, während andere Bereiche des Kühlelementes durch das Polstermaterial der Spielfigur abgeschirmt sind, so daß die Spielfigur vom Kind trotzdem noch als kuschelig und angenehm zum Anfassen beurteilt wird. Dadurch wird die Kälte des Kühlelementes, die vom Kind ansonsten als unangenehm empfunden wird, abgemildert.

Zweckmäßigerweise wird das Kühlfenster gemäß Anspruch 2 mit einem Textilmaterial abgedeckt, so daß zwar die Kälte durchtreten kann, eine Berührung des meist aus hartem Kunststoff bestehenden Kühlelementes unmittelbar mit der Haut des Kindes jedoch vermieden wird.

Zweckmäßigerweise wird das Kühlelement in einer extra dafür vorgesehenen Tasche gemäß den Ansprüchen 3 und 4 im Körper untergebracht.

In der Ausgestaltung nach Anspruch 5 kann das, üblicherweise ebene Flächen aufweisende, Kühlelement noch eine weitere Funktion als Stand- oder Sitzhilfe erfüllen.

Die Anordnung der Tasche gemäß Anspruch 6 gestattet einerseits, daß das Kühlelement schnell und einfache einsetz- bzw. auswechselbar ist, und erleichtert andererseits die Funktion des Kühlelementes als Sitz- oder Standhilfe.

Anspruch 7 beschreibt besonders bevorzugte Kühlelemente.

Ein Ausführungsbeispiel der Erfindung wird nachfolgend anhand der Zeichnungen näher erläutert. Es zeigen:
- Fig. 1: eine perspektivische Darstellung einer erfindungsgemäßen Spielfigur in Form eines Plüschbären, und
- Fig. 2: den Plüschbären aus Fig. 1 im teilweisen Längsschnitt.

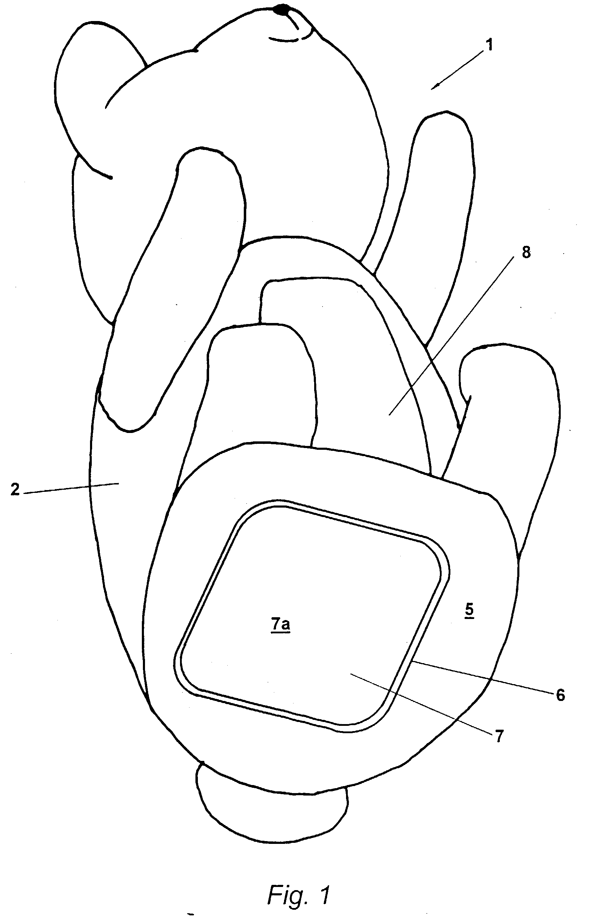

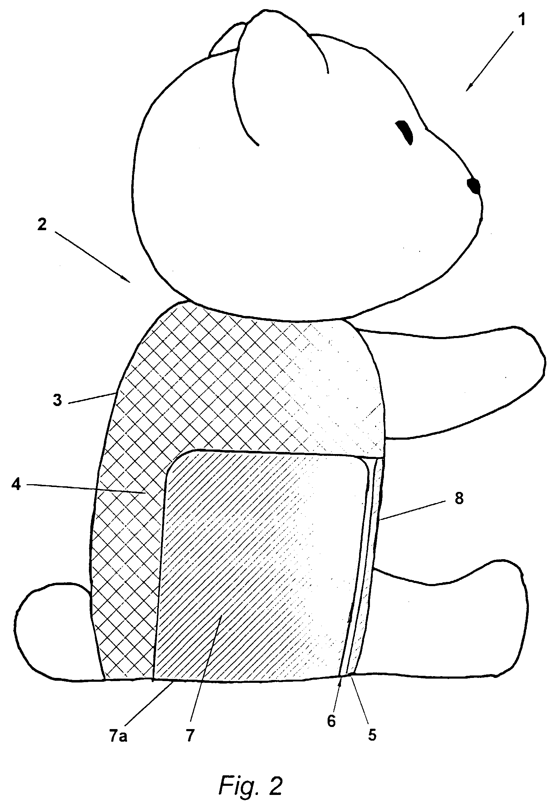

Aus den Fig. 1 und 2 ist ein erstes Ausführungsbeispiel einer erfindungsgemäßen Spielfigur 1 ersichtlich, die als Bär ausgebildet ist. Andere Tier- und Puppenformen bzw. gänzlich andere Ausgestaltungen sind möglich.

Die Spielfigur 1 enthält einen Körper 2, der aus einer Hülle 3 aus einem gewöhnlich für derartige Figuren verwendeten, textilen Plüschmaterial genäht wurde. Die Hülle 3 ist mit dem ebenfalls üblicherweise für derartige Zwecke eingesetzten Polstermaterial 4 ausgestopft. An einer als Sitzfläche 5 ausgebildeten Seite des Körpers 2 ist eine in das Innere des Körpers 2 hineinreichende Tasche 6 eingearbeitet, in der ein Kühlelement 7, dargestellt ist ein Kühlwürfel, gerade eben so hineinpaßt, daß er zwar leicht wieder entfernt werden kann, jedoch im Inneren der Tasche 6 durch Reibung am Textilmaterial der Tasche 6 gehalten wird. Die der Sitzfläche 5 anliegende Seite der Tasche 6 ist vollständig offen und das Kühlelement 7 ist so ausgebildet, daß es so weit in die Tasche 6 hineingeschoben werden kann, daß eine seiner ebenen Flächen 7a mit der Sitzfläche 5 fluchtet und somit als Sitzhilfe für den Bären dienen kann.

An einem Bereich der Tasche 6 ist ein Kühlfenster 8 vorgesehen. Das Kühlfenster 8 wird bevorzugt an einer Stelle der Spielfigur 1 angeordnet, die auch in Körperkontakt mit dem Kind steht, wenn das Kind die Spielfigur 1 an sich drückt. Im vorliegenden Ausführungsbeispiel ist dies der Bauch des Bären. Das Kühlfenster 8 enthält kein Polstermaterial, und ist allenfalls mit einer leichten Wattierung zur Versteifung versehen. Das Kühlfenster 8 kann beispielsweise lediglich aus dem Material der Hülle 3 gebildet werden, oder es kann ein farblich und/oder strukturmäßig abweichender Einsatz verwendet werden. Durch dieses Kühlfenster 8 wird zwar die Übertragung der Kälte vom Kühlelement 7 auf die entsprechenden Hautpartien des Kindes nicht behindert, eine unmittelbare Berührung mit dem Kühlelementjedoch vermieden. Dagegen werden diejenigen Bereiche, wie beispielsweise der Rumpf und der Kopf des Bären, die vom Kind normalerweise ergriffen werden, durch das Polstermaterial 4 gegen die Kälte des Kühlelementes 7 abgeschirmt.

Als Kühlelemente 7 können die handelsüblichen, mit einem Kühlmedium gefüllten, allseitig geschlossenen Behälter aus glattem Kunststoff eingesetzt weden. Wegen ihrer guten Handhabbarkeit und Eignung für die Zwecke der vorliegenden Erfindung sind würfel- oder quaderförmige Kühlelemente 7 bevorzugt.

In Abwandlung des beschriebenen und gezeichneten Ausführungsbeispiels kann die erfindungsgemäße Spielfigur als beliebiges Tier oder auch als Puppe oder dgl. eingesetzt werden. Bei Tieren, beispielsweise einem Pinguin, die normalerweise stehen, kann die Tasche an der Standfläche offen bleiben, so daß eine ebene Fläche des Kühlelementes als Standfläche dient. Falls erforderlich, kann die Tasche 6 auch durch beispielsweise eine Klappe oder dgl. geschlossen werden.

## Patentansprüche

1. Spielfigur, insbesondere Plüschfigur, mit einem mit Polstermaterial (4) gefüllten Körper (2), einem im Körper (2) untergebrachten Kühlelement (7) und einem im wesentlichen polstermaterialfreien Kühlfenster (8), das an einer Seite des Kühlelementes (7) vorgesehen ist.

2. Spielfigur nach Anspruch 1, **dadurch gekennzeichnet,** daß das Kühlfenster (8) mit Textilmaterial abgedeckt ist.

3. Spielfigur nach Anspruch 1 oder 2, **dadurch gekennzeichnet,** daß das Kühlelement (7) auswechselbar in einer im Körper (2) vorgesehenen Tasche (6) untergebracht ist.

4. Spielfigur nach Anspruch 3, **dadurch gekennzeichnet,** daß die Tasche (6) eine Zugriffsöffnung aufweist, die im Abstand zum Kühlfenster (8) angeordnet ist.

5. Spielfigur nach einem der Ansprüche 1 bis 4, **dadurch gekennzeichnet,** daß das Kühlelement (7) wenigstens eine ebene Fläche (7a) aufweist, die derart an einer Sitz- oder Stehfläche (5) des Körpers (2) angeordnet ist, daß das Kühlelement (7) als Sitz- oder Standhilfe dient.

6. Spielfigur nach einem der Ansprüche 3 bis 5, **dadurch gekennzeichnet,** daß die Tasche (6) an der Sitz- oder Stehfläche (5) nach außen offen ist.

7. Spielfigur nach einem der Ansprüche 1 bis 6, **dadurch gekennzeichnet,** daß das Kühlelement (7) ein geschlossener, ein Kältemedium umschließender Körper, insbesondere ein Würfel oder Quader ist.
